# EUROPEAN PATENT SPECIFICATION

(11) **EP 4 629 990 B1**
(45) Date of publication and mention of the grant of the patent: **19.08.2026**
(21) Application number: 23825469.2
(22) Date of filing: 06.12.2023
(51) Int. Cl.: A61K 31/437, A61P 5/28, A61P 13/08, A61P 17/14

(54) **PHENYL-PYRAZOLO[3,4-B]PYRIDINE-4-CARBOXYLIC ACID DERIVATES FOR USE AS 5-ALPHA REDUCTASE ANTAGONISTS IN METHODS OF TREATMENT**
PHENYLPYRAZOLO[3,4-B PYRIDIN-4-CARBONSÄUREDERIVATE ZUR VERWENDUNG ALS 5-ALPHA REDUKTASE ANTAGONISTEN IN BEHANDLUNGSMETHODEN
DÉRIVÉS D'ACIDE PHÉNYL-PYRAZOLO[3,4-B]PYRIDINE-4-CARBOXYLIQUE DESTINÉS À ÊTRE UTILISÉS EN TANT QU'ANTAGONISTES DE 5-ALPHA RÉDUCTASE DANS DES MÉTHODES DE TRAITEMENT

(30) Priority: 06.12.2022 GB 202218325
(43) Date of publication of application: 15.10.2025
(73) Proprietor: University Of Cape Town, 7700 Cape Town (ZA)
(72) Inventor: AROWOLO, Afolake, 7700 Cape Town (ZA); KHUMALO, Nonhlanhla Patience, 7700 Cape Town (ZA); OPUTU, Ogheneochuko Utieyin, 7700 Cape Town (ZA); NTSHINGILA, Sincengile Nokubonga, 7700 Cape Town (ZA); FIENBERG, Stephen, 7700 Cape Town (ZA)
(74) Representative: Icosa
(86) International application number: PCT/IB2023/062307
(87) International publication number: WO 2024/121771

(56) References cited:
- WO-A1-2013/039988
- WO-A1-2016/073956
- WO-A1-2018/206078
- STN: "Compound of CAS Registry Number 1160246-24-1 entered into STN 29June2009", 29 June 2009 (2009-06-29), STN, XP093131142, Retrieved from the Internet <URL:http://www.cas.org> [retrieved on 20240214]

## Description

### INTRODUCTION

This invention relates to compounds that are phenyl-pyrazolo[3,4-b]pyridine-4-carboxylic acid derivates for use as 5-alpha reductase antagonists. The invention further provides for pharmaceutical compositions comprising these compounds, and the use of these compounds and compositions in the treatment of diseases or disorders, in particular but not exclusively, in the treatment of androgenetic alopecia and benign prostatic hyperplasia.

### BACKGROUND

Androgenetic alopecia ("AGA"), commonly known as male pattern baldness, is a highly prevalent condition across all populations, predominantly affecting men and, to a lesser extent, women. AGA is non-cicatricial and mediated by follicular sensitivity to androgens and the individual's genetic predisposition. Though not fatal, it has potential adverse psychosocial sequelae and can decrease the quality of life.

AGA is mediated by the conversion of testosterone by 5-alpha reductase, type 2 (SRD5A2), into dihydrotestosterone (DHT), a more potent androgen that binds to androgen receptors with higher affinity. The upregulation of DHT results in perifollicular miniaturisation, inflammation, and fibrosis, which are hallmarks of AGA, eventually leading to hair loss in the scalp's AGA-sensitive sites. In addition to its role in AGA, predominant DHT in the prostate from 5-alpha reductase, type 1 (SRD5A1) plays a beneficial role in the development of benign prostate enlargement, which may later lead to prostate cancer. It is proposed in the literature that SRD5A1 and SRD5A2 is responsible for the production of one-third and two-thirds of circulating DHT, respectively.

Current treatment strategies for these DHT mediated conditions includes three Food and Drug Administration approved drugs. AGA is treated with minoxidil, a vasodilator and finasteride, a 5-alpha reductase, type 2 (SRD5A2) inhibitor. Benign prostatic hyperplasia ("BPH") is treated with Dutasteride, a 5-alpha reductase, type 1 (SRD5A1) inhibitor and finasteride which can also inhibit SRD5A1. However, finasteride is 100-fold more selective for SRD5A2 than SRD5A1. Also, minoxidil and finasteride are believed to be more effective when combined, and have to be taken for a minimum of four to six months to realise any substantial improvement and used over extended periods. While prolonged drug administration is a prerequisite for enhanced treatment response, it often leads to poor medication adherence and other adverse effects that persist beyond suspension of the drugs. Therefore, the need for AGA's alternate therapeutics, specifically on improved drug compounds that target SRD5A2, necessitates more research on novel efficacious drug molecules with minimal drawbacks or side effects.

SRD5A2 is a G-Protein Coupled Receptor ("GPCR)", with a recently solved structure comprising of the typical seven transmembranes (TM) domain helices. GPCRs represent the largest protein family encoded by the human genome, and constitute the primary therapeutic target for over 50% of currently marketed drugs. These molecules can bind to GPCRs as either agonists or antagonists. Agonists are drugs that bind to receptors, producing a similar response to the native substrate, upregulating the cellular response. Antagonist drugs bind to the receptor either on the orthosteric or allosteric site, inhibiting the receptor response.

The SRD5A2 active site has six cytosolic loops (L) and two separate tunnel-like pockets for NADP and DHF. The ligands' entry point to the binding site is between TM1 and TM4. Loop 1 (L1) controls the NADPH/NADP+ exchange from the cytosol. There are two catalytic residues, E57^{TM2} and Y91^{TM3}, in the active site responsible for reducing testosterone into DHT. These residues facilitate the hydride transfer from NADPH to testosterone (E57^{TM2} and Y91^{TM3}) The hydride transfer to the C-5 atom of testosterone forms an enolised intermediate followed by the reduction of the Δ^{4,5} bond in the testosterone, releasing DHT and NADP+.

The mechanism of inhibition for SRD5A2 consists of the hydride transfer from NADPH (4-pro-(R)-hydride) to the Δ^{1,2} bond in finasteride to form an enolised adduct of NADPH and finasteride (NADP-DHF). This covalent bond between NADP-DHF prevents the hydride transfer from NADPH to testosterone, and thus preventing the formation of DHT. The covalent NADP-DHF adduct has a dissociation constant Ki = koff/kon ≤ 1 × 10⁻¹³ M and ranks amongst the most potent noncovalently-bound inhibitors known for any enzyme. The protein interacts with the adduct through three key residues- E57, R114, and F118. E57^{TM2} interacts with finasteride, while residues R114 and F118 interact with the NADP moiety of NADP-DHF.

Finasteride acts as a competitive and specific inhibitor of SRD5A1 and 2, preventing the conversion of testosterone into the more active metabolite, 5-alpha dihydrotestosterone. However, the use of systemic finasteride has been associated with severe side effects.

Accordingly, it is therefore an object of the present invention to provide for alternative therapeutics that inhibit 5-alpha reductase, with improved selectivity and specificity.

It is a further object of the present invention to provide for new therapeutics that has improved selectively for the inhibition of 5-alpha reductase, in particular 5-alpha reductase type 2. WO2018206078A1 teaches that Finasteride can be used to treat benign prostatic hyperplasia or androgenic alopecia by DHT reduction and is a 5-alpha reductase inhibitor. WO2016073956A1 and WO2013039988A1 also teach that alternative compounds reduce DHT to treat benign prostatic hyperplasia or androgenic alopecia. CAS registry discloses a compound of the examples of the application, referred to as Bsy1.

### SUMMARY OF THE INVENTION

The invention is defined in the appended set of claims. Any references in the description to methods of treatment refer to the compounds, pharmaceutical compositions and medicaments of the present invention for use in a method for treatment of the human (or animal) body by therapy (or for diagnosis).

According to a first aspect to the present invention there is a pharmaceutical composition comprising a compound of the Formula (I), or a pharmaceutically acceptable salt, hydrate, or solvate thereof, and a pharmaceutically acceptable carrier, 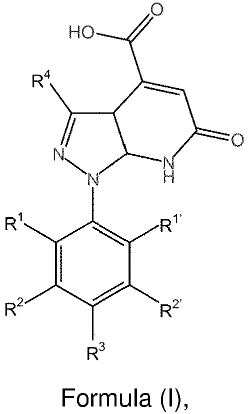 wherein,
R¹, R^{1'}, R², R^{2'}, and R³ are each independently selected from the group consisting of H, halogen, and -OR⁵,
R⁵ is selected from substituted or unsubstituted C₁-C₂alkyl, substituted or unsubstituted, linear or branched C₃-C₆alkyl, and
R⁴ is selected from substituted or unsubstituted C₁-C₂alkyl, substituted or unsubstituted, linear or branched C₃-C₆alkyl, and substituted or unsubstituted C₃-C₆cycloalkyl.

In one embodiment, R¹, R^{1'}, R², and R^{2'} are H, and R³ is -OR⁵.

In one embodiment, one of R¹ or R^{1'} is -OR⁵, and R², R^{2'}, and R³ are H.

In one embodiment, one of R² or R^{2'} is -OR⁵, and R¹, R^{1'}, and R³ are H.

In one embodiment, at least one of R¹, R^{1'}, R², R^{2'}, and R³ are halogen.

In one embodiment, one of R¹, R^{1'}, R², R^{2'}, and R³ is halogen and the rest if H.

In a further embodiment of the invention, R⁴ is selected from unsubstituted C₁-C₂alkyl, unsubstituted, linear or branched C₃-C₆alkyl, and unsubstituted C₃-C₆cycloalkyl.

According to a second aspect to the present invention there is provided for the use of a compound or a pharmaceutical composition of the invention as a medicament.

According to a third aspect to the present invention there is provided for the use of a compound or a pharmaceutical composition of the invention in a method of treating a disease or a disorder, the method comprising administering a pharmaceutically effective amount of the compound or composition to a subject in need thereof.

According to a further aspect of the present invention there is provided for the use of a compound of the invention in the preparation of a medicament for treating a disease or a disorder, the treatment comprising administering a pharmaceutically effective amount of the medicament to a subject in need thereof.

According to yet a further aspect of the present invention there is provided for a method of treating a disease or a disorder in a patient, the method comprising administering a compound or a pharmaceutical composition of the invention to a subject in need thereof.

In one embodiment, the disease or disorder is a disease or disorder mediated by high levels of dihydrotestosterone.

In one embodiment, the disease or disorder is benign prostatic hyperplasia or androgenic alopecia.

In a preferred embodiment, the disease or disorder is benign prostatic hyperplasia.

In a preferred embodiment, the disease or disorder is androgenic alopecia.

In a preferred embodiment, the subject is a human.

### BRIEF DESCRIPTION OF THE DRAWINGS

The invention will now be described in more detail with reference to the following non-limiting embodiments and figures in which:
- **Figure 1**: shows poses and intermolecular interactions for compound **Bsy1;**
- **Figure 2**: shows the relative expression (to HaCaT) of SRD5A genes in various cell lines (SRD5A2 gene is expressed in all the cell lines; SRD5A2 highly expressed in HepG2, HE293 and DU-145);
- **Figure 3**: shows DU-145 - drug IC₅₀ hyperbola curves. IC₅₀ values: Finasteride (0.72 ± 1.09 nM); Dutasteride (1.67 ± 1.85 µM); **Bsy1** (0.01 ± 0.015 nM);
- **Figure 4**: shows HEK293 - drug IC₅₀ hyperbola curves. IC₅₀ values: Finasteride (1.24 ± 1.43 nM); Dutasteride (1.21 ± 1.37 µM); **Bsy1** (0.02 ± 0.03 nM);
- **Figure 5**: shows the relative expression of the SRD5A gene after treatment of DU-145 cell line with Finasteride (0.9 nM), Dutasteride (1.6 µM) and **Bsy1** (0.0054 nM) at IC₅₀ values; and
- **Figure 6**: shows the relative expression of the SRD5A gene after treatment of HEK293 cell line with IC₅₀ values of Finasteride (1.34 nM), Dutasteride (1.29 µM) and **Bsy1** (0.02 nM).

### DETAILED DESCRIPTION OF A PREFERRED EMBODIMENT

The present invention will now be described more fully hereinafter with reference to the accompanying figures,

Although specific terms are employed herein, they are used in a generic and descriptive sense only and not for purposes of limitation.

As used herein, throughout this specification and in the claims which follow, the singular forms "a", "an" and "the" include the plural form, unless the context clearly indicates otherwise.

The terminology and phraseology used herein is for the purpose of description and should not be regarded as limiting. The use of the terms "comprising", "containing", "having", "including", and variations thereof used herein, are meant to encompass the items listed thereafter, and equivalents thereof as well as additional items.

When describing the invention, which includes pharmaceutical compositions including specific compounds and methods of using such compounds and compositions, the following terms, if present, have the following meanings, unless otherwise indicated. It should also be understood that when described herein any of the moieties defined forth below may be substituted with a variety of substituents, and that the respective definitions are intended to include such substituted moieties within their scope as set out below. In this regard, unless otherwise stated, the term "substituted" is to be defined as set out below. It should be further understood that the terms "groups" and "radicals" can be considered interchangeable when used herein.

"Alkyl" means straight or branched aliphatic hydrocarbon with the number of carbon atoms specified. Particular alkyl groups have 1 to 6 carbon atoms. More particular is lower alkyl which has 1 to 4 carbon atoms. A further particular group has 1 to 3 carbon atoms. Exemplary straight chain groups include methyl, ethyl *n*-propyl, and *n*-butyl. Branched means that one or more lower alkyl groups such as methyl, ethyl, propyl or butyl is attached to a linear alkyl chain. Exemplary branched chain groups include isopropyl and iso-butyl.

"Alkoxy" refers to the group -OR⁵, for example, wherein R⁵ is alkyl with the number of carbon atoms specified. Particular alkoxy groups are methoxy, ethoxy, *n*-propoxy, isopropoxy, *n*-butoxy, tert-butoxy, sec-butoxy, *n*-pentoxy, *n-*hexoxy, and 1,2-dimethylbutoxy. Particular alkoxy groups are lower alkoxy, *i.e.* with between 1 and 6 carbon atoms. Further particular alkoxy groups have between 1 and 4 carbon atoms.

"Alkylene" refers to divalent alkene radical groups having the number of carbon atoms specified, in particular having 1 to 6 carbon atoms and more particularly 1 to 4 carbon atoms which can be straight-chained or branched. This term is exemplified by groups such as methylene (-CH₂-), ethylene (-CH₂-CH₂-), or - CH(CH₃)- and the like.

"Alkenyl" refers to monovalent olefinically unsaturated hydrocarbon groups with the number of carbon atoms specified. Particular alkenyl has 2 to 8 carbon atoms, and more particularly, from 2 to 6 carbon atoms, which can be straight-chained or branched and having at least 1 and particularly from 1 to 2 sites of olefinic unsaturation. Particular alkenyl groups include ethenyl (-CH=CH₂), *n-*propenyl (-CH₂CH=CH₂), isopropenyl (-C(CH₃)=CH₂) and the like.

"Aryl" refers to a monovalent aromatic hydrocarbon group derived by the removal of one hydrogen atom from a single carbon atom of a parent aromatic ring system. In particular aryl refers to an aromatic ring structure, monocyclic or polycyclic, with the number of ring atoms specified. Specifically, the term includes groups that include from 6 to 10 ring members. Where the aryl group is a monocyclic ring system it preferentially contains 6 carbon atoms. Particularly aryl groups include phenyl, and naphthyl. The term "phenyl" and "Ph" is used interchangeably herein, unless indicated otherwise.

"Cycloalkyl" refers to a non-aromatic hydrocarbyl ring structure, monocyclic or polycyclic, with the number of ring atoms specified. A cycloalkyl may have from 3 to 10 carbon atoms, and in particular from 3 to 6 carbon atoms. Such cycloalkyl groups include, by way of example, single ring structures such as cyclopropyl, cyclobutyl, cyclopentyl, and cyclohexyl.

"Halo" or "halogen" refers to fluoro (F), chloro (CI), bromo (Br) and iodo (I). Particular halo groups are fluoro, bromo or chloro.

"Hetero" when used to describe a compound or a group present on a compound means that one or more carbon atoms in the compound or group have been replaced by a nitrogen, oxygen, or sulfur heteroatom. Hetero may be applied to any of the hydrocarbyl groups described above such as alkyl, *e.g.* heteroalkyl, cycloalkyl, *e.g.* heterocycloalkyl, aryl, *e.g.* heteroaryl, and the like having from 1 to 4, and particularly from 1, 2 or 3 heteroatoms, more typically 1 or 2 heteroatoms, for example a single heteroatom.

"Heteroaryl" means an aromatic ring structure, monocyclic or fused polycyclic, that includes one or more heteroatoms independently selected from O, N and S and the number of ring atoms specified. In particular, the aromatic ring structure may have from 5 to 9 ring members. The heteroaryl group can be, for example, a five membered or six membered monocyclic ring or a fused bicyclic structure formed from fused five and six membered rings or two fused six membered rings or, by way of a further example, two fused five membered rings. Each ring may contain up to four heteroatoms typically selected from nitrogen, sulphur and oxygen. Typically, the heteroaryl ring will contain up to 4 heteroatoms, more typically up to 3 heteroatoms, more usually up to 2, for example a single heteroatom. In one embodiment, the heteroaryl ring contains at least one ring nitrogen atom. The nitrogen atoms in the heteroaryl rings can be basic, as in the case of an imidazole or pyridine, or essentially non-basic as in the case of an indole or pyrrole nitrogen. In general, the number of basic nitrogen atoms present in the heteroaryl group, including any amino group substituents of the ring, will be less than five.

As used herein, the term "heterocycloalkyl" means a stable non-aromatic ring structure, monocyclic or polycyclic, that includes one or more heteroatoms independently selected from O, N and S. The non-aromatic ring structure may have from 4 to 10 ring members, and in particular from 4 to 6 ring members. A fused heterocyclic ring system may include carbocyclic rings and need only to include one heterocyclic ring. As used herein, the term "heterocycloalkenyl" means a "heterocycloalkyl", wherein one bond of the ring is reduced, thus the ring comprises a double bond.

"Substituted" refers to a group in which one or more hydrogen atoms are each independently replaced with the same or different substituent(s).

As used herein, term "substituted with one or more" refers to one to four substituents. In one embodiment it refers to one to three substituents. In further embodiments it refers to one or two substituents. In a yet further embodiment, it refers to one substituent.

"Pharmaceutically acceptable" means approved or approvable by a regulatory agency such as the United States Food and Drug Administration agency, or any similar agency in countries other than the United States, or that is listed in a generally recognized pharmacopoeia for use in animals, and more particularly in humans, such as the U.S. Pharmacopoeia.

"Pharmaceutically acceptable salt" refers to a salt of a compound of the invention that is pharmaceutically acceptable and that possesses the desired pharmacological activity of the parent compound. In particular, such salts are non-toxic may be inorganic or organic acid addition salts and base addition salts. Specifically, such salts include: (1) acid addition salts, formed with inorganic acids including: hydrochloric acid, hydrobromic acid, sulfuric acid, nitric acid, and phosphoric acid; or formed with organic acids including: acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl)

benzoic acid, cinnamic acid, mandelic acid, methanesulfonic acid, ethanesulfonic acid, 1,2-ethane-disulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, 4-methylbicyclo[2.2.2]-oct-2-ene-1-carboxylic acid, glucoheptonic acid, 3-phenylpropionic acid, trimethylacetic acid, tertiary butylacetic acid, lauryl sulfuric acid, gluconic acid, glutamic acid, hydroxynaphthoic acid, salicylic acid, stearic acid, and muconic acid; or (2) salts formed when an acidic proton present in the parent compound either is replaced by a metal ion, *e.g*. an alkali metal ion, an alkaline earth ion, or an aluminum ion; or coordinates with an organic base including ethanolamine, diethanolamine, triethanolamine, and N-methylglucamine. Salts further include, by way of example only, sodium, potassium, calcium, magnesium, ammonium, and tetraalkylammonium; and when the compound contains a basic functionality, salts of non-toxic organic or inorganic acids, including hydrochloride, hydrobromide, tartrate, mesylate, acetate, maleate, and oxalate. The term 'pharmaceutically acceptable cation' refers to an acceptable cationic counter-ion of an acidic functional group. Such cations are exemplified by sodium, potassium, calcium, magnesium, ammonium, and tetraalkylammonium cations.

"Pharmaceutically acceptable vehicle" refers to a diluent, adjuvant, excipient or carrier with which a compound of the invention is administered.

"Solvate" refers to forms of the compound that are associated with a solvent, usually by a solvolysis reaction. This physical association includes hydrogen bonding. Conventional solvents include, by way of example, water, ethanol, and acetic acid. The compounds of the invention may be prepared, for example, in crystalline form and may then be solvated or hydrated. Suitable solvates include pharmaceutically acceptable solvates, such as hydrates, and further include both stoichiometric solvates and non-stoichiometric solvates. In certain instances, the solvate will be capable of isolation, for example when one or more solvent molecules are incorporated in the crystal lattice of the crystalline solid. "Solvate" encompasses both solution-phase and isolable solvates. Representative solvates include hydrates, ethanolates and methanolates.

"Subject" includes humans. The terms "human", "patient" and "subject" are used interchangeably herein.

"Effective amount" means the amount of a compound of the invention that, when administered to a subject for treating a disease, is sufficient to effect such treatment for the disease. The "effective amount" can vary depending on the compound, the disease and its severity, and the age, weight, etc., of the subject to be treated.

"Treating" or "treatment" of any disease or disorder includes ameliorating the disease or disorder, *i.e.* arresting the disease or reducing the manifestation, extent or severity of at least one of the clinical symptoms thereof. In another embodiment "treating" or "treatment" refers to ameliorating at least one physical parameter, which may not be discernible by the subject. In yet another embodiment, "treating' or 'treatment" refers to modulating the disease or disorder, either physically, (*e.g*. stabilization of a discernible symptom), physiologically, (*e.g*. stabilization of a physical parameter), or both. In a further embodiment, "treating" or "treatment" relates to slowing the progression of the disease.

Where ranges are referred to in this specification, for example C₁₋₄ alkyl, the citation of a range should be considered a representation of each member of the range.

It is also to be understood that compounds that have the same molecular formula but differ in the nature or sequence of bonding of their atoms or the arrangement of their atoms in space are termed "isomers". Isomers that differ in the arrangement of their atoms in space are termed "stereoisomers".

"Stereoisomers" that are not mirror images of one another are termed "diastereomers" and those that are non-superimposable mirror images of each other are termed "enantiomers". When a compound has an asymmetric center, for example, it is bonded to four different groups, a pair of enantiomers is possible. An enantiomer can be characterized by the absolute configuration of its asymmetric center and is described by the *R*- and *S*- sequencing rules of Cahn and Prelog, or by the manner in which the molecule rotates the plane of polarized light and designated as dextrorotatory or levorotatory (*i.e.* as (+) or (-)-isomers respectively). A chiral compound can exist as either individual enantiomer or as a mixture thereof. A mixture containing equal proportions of the enantiomers is called a "racemic mixture"'.

"Tautomers" refer to compounds that are interchangeable forms of a particular compound structure, and that vary in the displacement of hydrogen atoms and electrons. Thus, two structures may be in equilibrium through the movement of π electrons and an atom (usually H). For example, enols and ketones are tautomers because they are rapidly interconverted by treatment with either acid or base. Another example of tautomerism is the aci- and nitro- forms of phenylnitromethane, that are likewise formed by treatment with acid or base. Tautomeric forms may be relevant to the attainment of the optimal chemical reactivity and biological activity of a compound of interest.

The compounds of the invention may possess one or more asymmetric centers; such compounds can therefore be produced as individual (*R*)- or (*S*)-stereoisomers or as mixtures thereof. Unless indicated otherwise, the description or naming of a particular compound in the specification and claims is intended to include both individual enantiomers and mixtures, racemic or otherwise, thereof. The methods for the determination of stereochemistry and the separation of stereoisomers are well-known in the art.

It will be appreciated by those skilled in the art that compounds of the invention may be metabolized to yield biologically active metabolites.

The present invention provides for compounds that are phenyl-pyrazolo[3,4-b]pyridine-4-carboxylic acid derivates for use as 5-alpha reductase antagonists, in particular as type 1 and type 2 antagonists. The invention further provides for pharmaceutical compositions comprising these compounds, and the use of these compounds and compositions in the treatment of diseases or disorders, in particular but not exclusively, in the treatment of benign prostatic hyperplasia and androgenetic alopecia.

According to a first aspect to the present invention there is a pharmaceutical composition comprising a compound of the Formula (I), or a pharmaceutically acceptable salt, hydrate, or solvate thereof, and a pharmaceutically acceptable carrier, 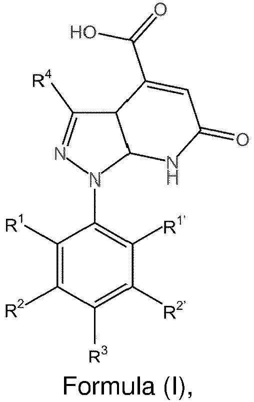 wherein,
R¹, R^{1'}, R², R^{2'}, and R³ are each independently selected from the group consisting of H, halogen, and -OR⁵,
R⁵ is selected from substituted or unsubstituted C₁-C₂alkyl, substituted or unsubstituted, linear or branched C₃-C₆alkyl, and
R⁴ is selected from substituted or unsubstituted C₁-C₂alkyl, substituted or unsubstituted, linear or branched C₃-C₆alkyl, and substituted or unsubstituted C₃-C₆cycloalkyl.

In one embodiment of the invention, R¹, R^{1'}, R², and R^{2'} are H, and R³ is -OR⁵.

In one embodiment, the one of R¹ or R^{1'} is -OR⁵, and R², R^{2'}, and R³ are H.

In one embodiment, the one of R² or R^{2'} is -OR⁵, and R¹, R^{1'}, and R³ are H.

In one embodiment, the at least one of R¹, R^{1'}, R², R^{2'}, and R³ are halogen.

In one embodiment, the one of R¹, R^{1'}, R², R^{2'}, and R³ is halogen and the rest if H.

In a further embodiment of the invention, R⁴ is selected from unsubstituted C₁-C₂alkyl, unsubstituted, linear or branched C₃-C₆alkyl, and unsubstituted C₃-C₆cycloalkyl.

Exemplary compounds included in the pharmaceutical compositions of the present invention may be represented by the chemical structures provided in the Table 1 below.

**Table 1. Chemical structures of exemplary compounds of the Formula (I).**

| **Compound** | **Chemical structure** | **Compound name** |
|---|---|---|
| **Bsy1** | 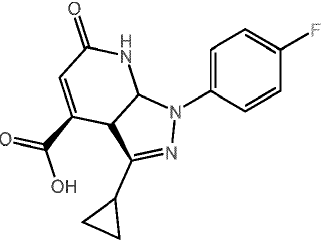 | 3-cyclopropyl-1-(4-fluorophenyl)-6-oxo-3a,6,7,7a-tetrahydro-1H-pyrazolo[3,4-b]pyridine-4-carboxylic acid |
| **Bsy2** | 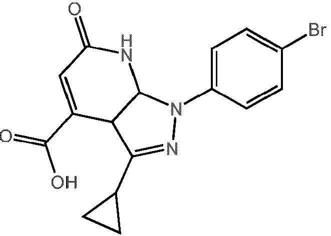 | 1-(4-bromophenyl)-3-cyclopropyl-6-oxo-3a,6,7,7a-tetrahydro-1H-pyrazolo[3,4-b]pyridine-4-carboxylic acid |
| **Bsy3** | 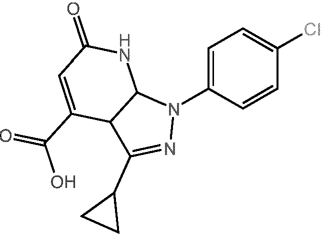 | 1-(4-chlorophenyl)-3-cyclopropyl-6-oxo-3a,6,7,7a-tetrahydro-1H-pyrazolo[3,4-b]pyridine-4-carboxylic acid |
| **Bsy4** | 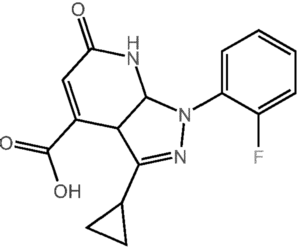 | 3-cyclopropyl-1-(2-fluorophenyl)-6-oxo-3a,6,7,7a-tetrahydro-1H-pyrazolo[3,4-b]pyridine-4-carboxylic acid |
| **Bsy5** | 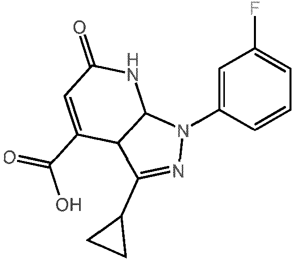 | 3-cyclopropyl-1-(3-fluorophenyl)-6-oxo-3a,6,7,7a-tetrahydro-1H-pyrazolo[3,4-b]pyridine-4-carboxylic acid |
| **Bsy6** | 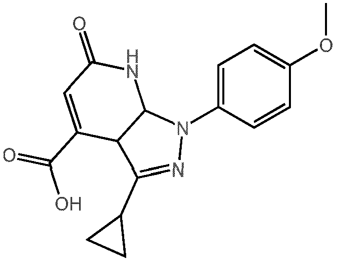 | 3-cyclopropyl-1-(4-methoxyphenyl)-6-oxo-3a,6,7,7a-tetrahydro-1H-pyrazolo[3,4-b]pyridine-4-carboxylic acid |
| **Bsy7** | 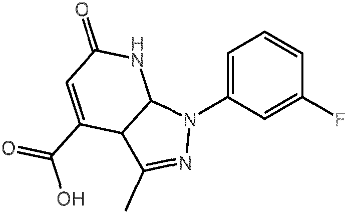 | 1-(3-fluorophenyl)-3-methyl-6-oxo-3a,6,7,7a-tetrahydro-1H-pyrazolo[3,4-b]pyridine-4-carboxylic acid |
| **Bsy8** | 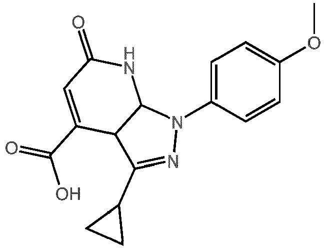 | 3-cyclopropyl-1-(4-methoxyphenyl)-6-oxo-3a,6,7,7a-tetrahydro-1H-pyrazolo[3,4-b]pyridine-4-carboxylic acid |
| **Bsy9** | 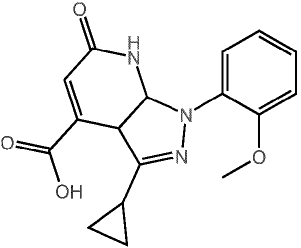 | 3-cyclopropyl-1-(2-methoxyphenyl)-6-oxo-3a,6,7,7a-tetrahydro-1H-pyrazolo[3,4-b]pyridine-4-carboxylic acid |
| **Bsy10** | 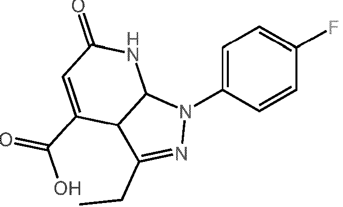 | 3-ethyl-1-(4-fluorophenyl)-6-oxo-3a,6,7,7a-tetrahydro-1H-pyrazolo[3,4-b]pyridine-4-carboxylic acid |

### 1. In silico target identification, ligand preparation, docking, and toxicity screening

### 1.1 Target identification and validation

The recently resolved structure of SRD5A2 was co-crystallised with a dual steric ligand, modelled as an adduct of finasteride and NADP (NADP-DHF). Analysis of this protein structure guided the inventors in the identification and selection of compounds with potential for further testing and analysis.

### 1.2 Ligand preparation and modelling

Following the finasteride binding mechanism, the structures of the selected compounds were drawn in a covalent bond complex with NADPH using the ChemDraw Professional software (Chemdraw RRID: SCR_016768), and these compounds were docked as the real ligand. The SRD5A2 mechanism of inhibition by finasteride is such that SRD5A2 catalyses a hydride transfer from NADPH to the C-5 atom of finasteride, leading to the formation of a covalent bond between the NADPH and finasteride.

The modelled compounds were prepared for computational study at physiological pH conditions using the LigPrep module of Schrödinger suite v 12.3 (Schrödinger, LLC, NY, USA, 2020). During preparation, Ligprep produced several structures from each input structure with various ionisation states, tautomers, stereochemistries, and ring conformations for each successfully processed input structure. The OPLS-4 force field was used to minimise the ligand geometry's optimisation and produce the ligand's low-energy conformer: creating 3-D geometries, assigning proper bond orders, and generating accessible tautomer and ionisation states before the screening. Epik, from the LigPrep module, was used to create ionisation/tautomeric states and calculate reasonable ligand states.

### 1.3 Target protein preparation

The coordinates and crystal structure of SRD5A2 were retrieved from the RCSB protein data bank (https://www.rcsb.org/) with the accession code 7BW1 at a 2.8-Å X-ray resolution. The 3D structure of SRD5A2 was prepared using the Protein Preparation Workflow Wizard (PPWW) in Maestro v 12.3 (Schrödinger, LLC, NY, USA, 2020). The protein minimisation was restrained for all-atom with a termination criterion based on the heavy atoms' root-mean-square deviation (RMSD= 0.3 Å) compared to their first location. Water molecules outside a 5 Å radius from ligands (hets) were removed.

### 1.4 Receptor grid generation

The Ligand Docking with Energetics (Glide) receptor grid generation wizard was used to generate the receptor grid using default settings (Van der Waals radius scaling of 1.0 Å with a partial charge cut-off of 0.25). The protein's active site was identified as the centroid of the workspace ligand pre-selected when defining the receptor. No constraints were defined during receptor grid generation, and only ligands similar in size to the workspace ligand were docked. The receptor grid for the plant compounds was generated slightly differently from the synthetic compounds grid. Two receptor grid models were developed: the first model had NADPH in the substrate-binding cavity treated as part of the protein, and the second model removed NADPH from the binding cavity.

### 1.5 Docking validation

The docking model was validated by re-docking the NADP-DHF adduct into the active site and ensuring that critical interactions between the receptor and ligand are maintained. A "CORE constraint" fixed the NADPH. The resultant pose of the re-docked ligand was superimposed with the co-crystalised ligand.

### 1.6 Molecular docking and screening of compounds

The selected compounds and two positive controls (finasteride and dutasteride) were docked into the binding site of SRD5A2 using the previously generated receptor grid. Molecular docking was performed using the Glide docking module of Maestro, with the OPLS-4 force field in the simulations against the curated libraries. The Glide docking was constrained at 0.3: and a "core" constraint was set when docking the compounds, fixing the NADP moiety of the adduct to ensure the docking poses have NADP in a fixed position. The 'Standard Precision' (SP-visualizer module) was used to read the glide score (G score), and the compounds were ranked based on their docking scores representing binding energies.

### 1.7 Binding energy prediction

The Prime MM-GBSA (Prime model of the Schrödinger suite 2021-4) was used to predict the binding energy of the bound ligands by rescoring a pre-generated ensemble of the SRD5A2 poses produced from docking. The MM-GBSA incorporated the OPLS-4 power field and a novel energy dissolvable model (VSGB) to run calculations, incorporating the generalised born surface area implicit solvation model. The Prime MM-GBSA module ranked and presented binding free energies for the docked compounds: highest negative value indicated the best-possible interaction.

From the 58 compounds selected for docking experiments, the Glide docking and MM-GBSA bind yielded eight compounds with good poses and conformations. One of these compounds, referred to herein as **Bsy1,** were selected from the eight compounds for further evaluation together with phenyl-pyrazolo[3,4-b]pyridine-4-carboxylic acid derivates of **Bsy1** referred to herein as **Bsy2** to **Bsy10** (see Table 1).

### 1.8 Toxicity prediction

ProTox-II (http://tox.charite.de/protox_II) was used to predict the toxicity and drug-like properties of the docked compounds.

Table 2 presents the selected compounds' predicted toxicity profiles and drug-like properties. The toxicity prediction of these compounds shows that the compounds belong to class 4, with an LD₅₀ of 418 mg/kg for **Bsy1.** Class 4 is harmful when swallowed according to GHS standards and must be administered in minimal doses. The Lipinski 'Rule of 5', a set of physicochemical parameters under which compounds are likely to make an excellent oral drug, was used to describe the drug-like properties of the compounds. Compound **Bsy1** did not violate the Lipinski rules and had a bioavailability score of 0.56, making it a viable candidate for therapeutics (bioavailability measured with SwissADME; http://www.swissadme.ch/).

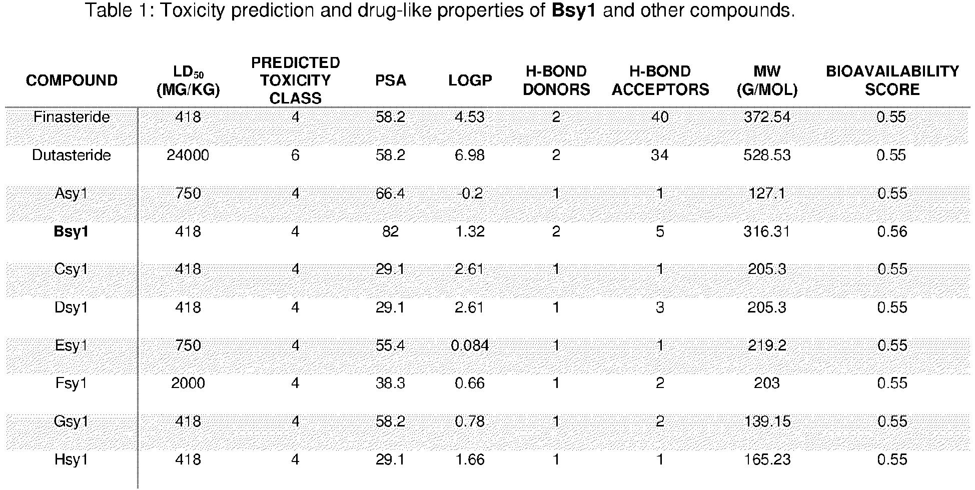

### 1.9 Docking results and binding free energy for synthetic compounds

The resultant output of the molecular docking of the original selection of 58 compounds yielded eight compounds within the glide score cut-off of ≤ -17 kcal/mol: the study visually inspected these compounds. Of the compounds that showed promise, compound **Bsy1** was selected for further evaluation and development at this time.

### 1.10 The protein-ligand complexes

Post-docking refinement and rescoring were done using Prime MMGBSA dG to describe the relative binding energies of each molecule (Table 3). The compounds had MM-GBSA dG bind scores ranging between -128.45 kcal/mol and - 157.73 kcal/mol.

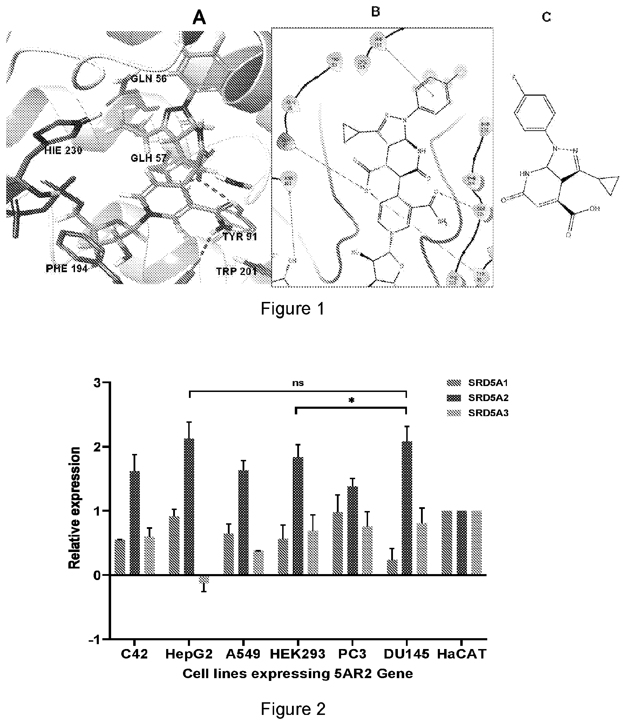

The binding mode and residue interaction pattern for compound **Bsy1** is shown in Figure 1. Figure 1A shows the binding pose and protein-ligand interaction (H-bond) with residues E57, Y91; and *ππ* interactions between F118 and the *p-*fluorophenyl group. Figure 1B shows 2-D intermolecular interactions. Figure 1C shows the 2-D chemical structure of compound **Bsy1. Bsy1** displays the interaction between residues E57 and Y91 (phenol group) via the carboxylate functional group of the reaction head. The binding force is due to O_{carboxyl}-H...O = C_{carboxylate} and O_{phenyl-}H...O_{carboxylate}. **Bsy1** also has a *ππ* stacking interaction with the p-fluorophenyl group (Figure 1 B).

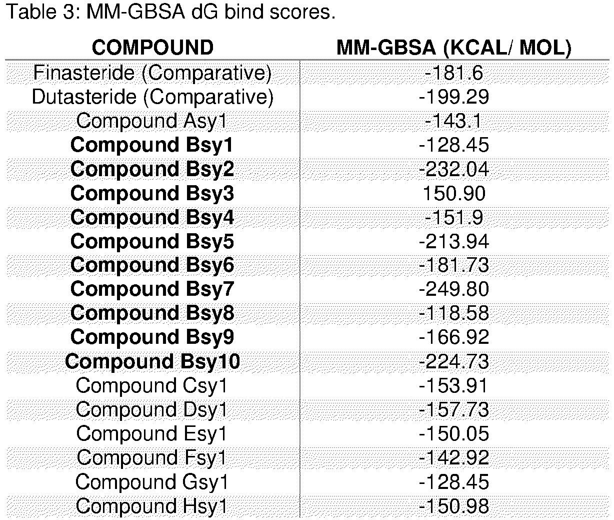

### 2. In-vitro screening and validation of Bsy1, and Bsy1 Derivates, as SRD5A2 Inhibitors

### 2.1 Evaluation of the relative expression levels of SRD5A2

Seven cell lines associated with tissues or organs expressing the endogenous SRD5A2 gene were selected, cultured to about 90% confluency and RNA extracted and the relative endogenous SRD5A2 gene expression levels of these cell lines quantified using the quantitative polymerase chain reaction (qPCR) technique. These were (i) C4-2, a human prostatic carcinoma cell line; (ii) HepG2, a human liver cancer cell line; (iii) A549, adenocarcinoma human alveolar basal epithelial cells; (iv) PC-3, and (v) DU-145, both human prostate cancer cell lines; (vi) HEK293, a human embryonic kidney cell line commonly used in recombinant expressions; and vii) HaCaTs, an immortalised human keratinocyte.

The qPCR results comparing the relative expression of the three SRD5A genes showed that the SRD5A2 gene was expressed about 2-fold in all the cell lines. The control cell lines were HaCaTs. The SRD5A2 gene was highly expressed in HepG2 (Liver), DU-145 (prostate), and HEK293 (human embryo) (Figure 2). The Mann-Whitney t-test statistical analysis compared the expression levels of the two cancer cell lines (HepG2 and DU-145). There was no statistically significant difference in the expression of SRD5A2 between HepG2 and DU-145 (p=0.6667), and the study selected DU-145 cell lines based on its extensive reporting for SRD5A2 cell-based assays. For selectivity purposes, the study used the cancer cell line (DU-145) and non-cancer cell line (HEK293) to screen compounds that inhibit SRD5A2.

### 2.2 Evaluation of the relative expression levels of SRD5A2 treated with the test compounds

The DU-145 cell lines and HEK293 cell lines were grown to 70% confluence in DMEM supplemented with 10 % (v/v) fetal bovine serum and 1% antibiotic (100 µg/mL of streptomycin and 100 unit/mL of penicillin (P/S) at 37 ºC in a 5% CO₂ environment.

The half maximal inhibitory concentration (IC₅₀) was established in the selected cell lines. The cell lines previously identified as having high endogenous expression levels of SRD5A2 were used to determine the *in-vitro* cytotoxic effects of **Bsy1** on DU-145 and HEK293. The results were compared with the comparative compounds finasteride and dutasteride. The cytotoxic concentration and cell viability of these drugs were evaluated using the 3-(4,5-dimethylthiazol-2-yl)-2,5-diphenyl-2H-tetrazolium bromide ("MTT") assay, using the comparative compounds as positive controls.

The study seeded cells at 1 × 10⁴ cells/well density using a 96-well plate and incubated them for 24 hours to allow the cells to attach in a 5% CO₂ incubator at 37 ºC. Different serial concentrations of the compounds treated the cells, 1% (v/v) DMSO/methanol in DMEM treated the control cells, and DMEM only served as a blank. Cells were stained with 100 µl of a 0.5 mg/ml MTT solution and incubated in a 5% CO₂ atmosphere at 37 ºC incubators for 4 hours. The medium was aspirated, and 100 µl of absolute ethanol was added to each well. The formation of tetrazolium salt measured all growth and chemosensitivity. Absorbance was detected spectrophotometrically at 570 nm using a reference wavelength of 630 nm. The experiments were performed in triplicate.

The dose-response from the non-linear curve (inhibitor vs normalised response - variable slope curve), plotted using GraphPad Prism V 9.3.1, was used to determine the IC₅₀ values of these cells.

The selectivity index, indicating the selectivity of a given compound between normal cells and cancer cells, was calculated from the ratio of their respective IC₅₀ values (Equation 1). $SI = \frac{IC 50 of a compound in normal cell line \left(HEK293\right)}{IC 50 of same compound in cancer cell line \left(DU-145\right)}$

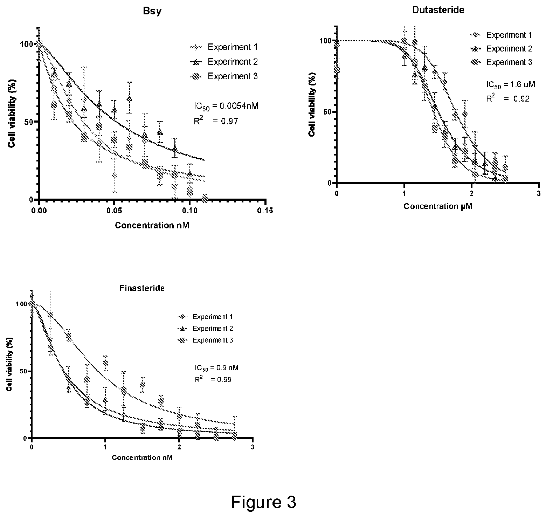

The dose-response curves for the DU-145 cell line revealed that **Bsy1** had an antagonist activity at 0.0054 nM (5.4 pM) against SRD5A2, compared to finasteride at 0.9 nM and dutasteride at 1.6 µM. The study determined the IC₅₀ values using a non-linear curve fit with a hyperbola model (Figure 3). The IC₅₀ values of the standard drugs were comparable to those obtained in other studies, suggesting the assay was suitable for determining the inhibitory kinetics and IC₅₀ values of **Bsy1.**

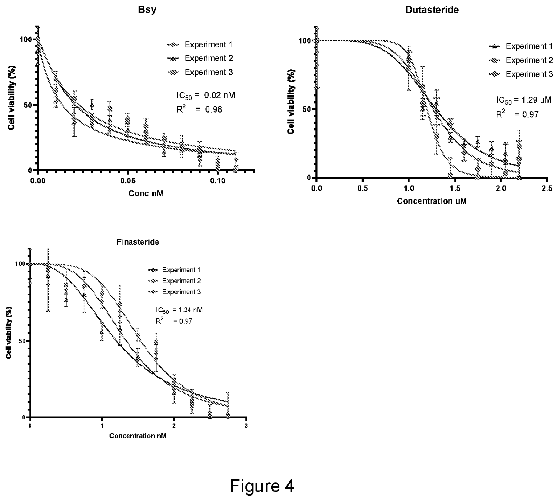

However, the IC₅₀ values for **Bsy1** in the HEK293 cell lines were higher than in the DU-145 cells. **Bsy1** had an antagonist activity at 0.02 nM against SRD5A2, compared to finasteride at 1.34 nM and dutasteride at 1.29 µM (Figure 4).

The IC₅₀ values of **Bsy1** in the DU-145 cancer cell lines were lower than the IC₅₀ values in the non-cancer HEK293 cell lines, which indicates higher potency of the compound on the cancer cell line. The HEK293 cell line was more resistant to the compounds, including **Bsy1,** than the DU-145 cell line, with a lower drug activity.

The estimated selectivity index (Table 3, equation 1) demonstrated differential activity of compound **Bsy1** at 3.7 units, which is considerably higher than finasteride (1.49 units) and dutasteride (0.81). It is generally accepted that values greater than 1 indicate desirable selectivity against cancer cells, and suggest potentially better toxicity profiles than those with a value less than 1.

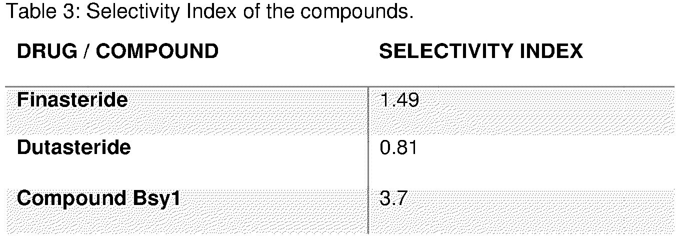

### 2.3 Relative expression of the SRD5A gene in DU-145 cell lines

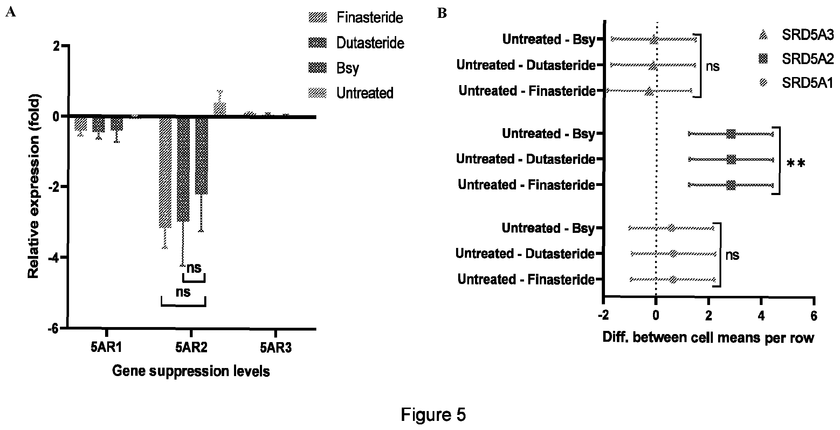

The androgen-independent DU-145 cell line, treated with finasteride (0.9 nM), dutasteride (1.6 µM), and **Bsy1** (0.0054 nM) for 24 hours, was measured for their SRD5A gene regulation using qPCR. The results showed that compared to the control (untreated), the tested compounds induced a statistically significant downregulation of SRD5A2 (p < 0.001) in the DU-145 cell line. The relative expression of the SRD5A2 gene was reduced by approximately 3-fold for finasteride and dutasteride and 2-fold for **Bsy1** (Figure 5A). However, it should be noted that the finasteride concentrations were 100-fold, and dutasteride 1000-fold, higher than the concentration of **Bsy1.** Also, as can be seen from Figure 5A, SRD5A1 suppression by finasteride and **Bsy1** was comparable even though finasteride concentrations were 100-fold that of **Bsy1.** The level of inhibitory action against SRD5A1 and SRD5A2 is surprising as it is expected that that the phenyl-pyrazolo[3,4-b]pyridine-4-carboxylic acid **Bsy1** and its derivates, as described herein, will show similar inhibition results. *[**Figure 5A* *- Relative expression of the SRD5A gene after treatment of DU-145 cell line with finasteride (0.9 nM), dutasteride (1.6* µ*M*) *and **Bsy1** (0.0054 nM) at IC₅₀ values. 'ns'- expression levels not statistically different between finasteride and compound **Bsy1;** dutasteride and compound **Bsy1.** Experiments were repeated three times.]*

A two-way ANOVA statistical analysis was conducted to compare the gene suppression levels of each drug treatment against the control (untreated), using Dunnett's correction test (DCT) (Figure 5B). *[Figure 5B* *- two-way ANOVA Dunnett test of the Control vs Treatment at IC₅₀ values. Comparison tests of control vs. drug treatment at p < 0.05 for: SRD5A1 (ns); SRD5A2**; SRD5A3 (ns). Data were expressed for each bar as means ± standard error of mean **P* ≤ *0.001 from control, #P ≤ 0.001. Significantly down-regulated genes are on the right of the zero margins with positive values on the graph and should not lie on the 'zero' line.]*

### 2.4 Relative expression of the SRD5A gene in HEK293 cell lines

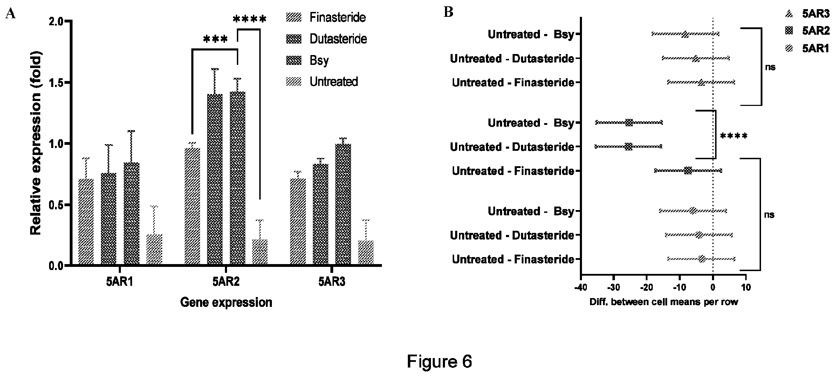

The HEK293 cell lines, treated with finasteride (1.34 nM); dutasteride (1.29 µM); compound **Bsy1** (0.02 nM) for 24 hours, were measured for their SRD5A gene regulation using qPCR (Figure 6A). The results showed that, compared to the control, the tested compounds induced a significant upregulation of SRD5A2 (p < 0.0001) in the HEK293 cell lines. Similar results were observed for SRD5A1 and SRD5A3. The relative expression of the SRD5A2 gene increased by approximately 2-fold for compound **Bsy1** and dutasteride; and 0.75-fold for finasteride. *[**Figure 6A* *- Relative expression of the SRD5A gene after treatment of HEK293 cell line with IC₅₀ values of finasteride (1.34 nM), dutasteride (1.29* µ*M) and **Bsy1** (0.02 nM). 'ns'- expression levels not statistically different between finasteride and compound **Bsy1;** dutasteride and compound **Bsy1.** Experiments were repeated three times.]*

The two-way ANOVA compared the expression levels of the SRD5A genes and between the different drugs in HEK293 cell lines (Figure 6 B). *[Figure 6B* *- two-way ANOVA Dunnett test of the Control vs Treatment at IC₅₀ values on HEK293 cell lines. Comparison tests of control vs. drug treatment at p < 0.05 for: SRD5A1 (ns); SRD5A2****, except for finasteride vs untreated (ns); SRD5A3 (ns). Data were expressed for each bar as means ± standard error of mean ****P* ≤ *0.0001 from control. Significantly upregulated genes are on the left of the zero margins, without touching the graph's zero line.]*

Compound **Bsy1** and the reference drugs (finasteride and dutasteride) were screened for SRD5A2 and SRD5A1 inhibition in DU-145 and HEK293 cell lines. Before the screening, the cells were treated with varying concentrations of the drugs to determine their IC₅₀ values, which ranged between 5.4 pM (0.0054 nM) and 1.2 µM. Of the tested compounds **Bsy1** had the lowest IC₅₀ of 5.4 pM in DU-145 and 0.02 nM in HEK293, with a selectivity index of 3.7, which is an indication of possible higher potency and higher resistance of normal cell line HEK293, *i.e.* greater selectivity. The reference drugs had a higher IC₅₀ than **Bsy1** on the cancer cell line than in the normal cell line, indicating the potentially low potency of these compounds compared to **Bsy1.** Based on the in-silico work, and the in vitro results obtained for **Bsy1,** it would be reasonable to expect that the phenyl-pyrazolo[3,4-b]pyridine-4-carboxylic acid derivates of **Bsy1,** including analogues **Bsy2** to **Bsy10** would show similar results, and therefore be viable therapeutic agents for the treatment of the androgen dependent disorders, benign prostatic hyperplasia and androgenic alopecia.

In the experiments conducted the cells were treated with IC₅₀ values of the drugs over 24 hours and determined the gene regulation levels of SRD5A2 and SRD5A1. The three compounds suppressed the SRD5A2 and SRD5A1 genes in the DU-145 cancer cell lines, while the genes were upregulated in the HEK293 normal cells. The relative expression of the SRD5A2 gene was downregulated by approximately 3-fold for finasteride and dutasteride and 2-fold for **Bsy1** for SRD5A2 in the DU-145 cells, with finasteride concentrations 100-fold that of **Bsy1.** However, it upregulated by approximately 2-fold for **Bsy1** and dutasteride and 0.75-fold for finasteride in the HEK293 cells.

### 3. In-vitro pharmacokinetics profiling of the drugs

The solubility (**Bsy1-6** and finasteride) and hepatocyte stability (**Bsy1** and finasteride) were tested. Solubility of a drug is an important parameter to achieve desired concentration of drug in systemic circulation for achieving required pharmacological response. Poorly water-soluble drugs often require high doses in order to reach therapeutic plasma concentrations after oral administration.

The hepatocyte stability test is important to measure the metabolism and clearance of drugs by the liver, absorbed through the gut. *In-vitro* metabolic stability is a good measure of the drugs *in-vivo* half-life and clearance of the test compound.

### 3.1 Evaluation of the solubility and hepatocyte stability of Bsy1-6 and finasteride

Solubility was measured at pH 6.5 using an adapted miniaturised shake-flask method, in 96-well plate format. Briefly, 4 µl of a 10mM stock in DMSO was added to a 96-well plate and evaporated using a GeneVac system. Phosphate buffer pH 6.5 was then added to the wells and the plate was incubated for 24 h at 25 °C with shaking. At the end of this incubation, the samples were centrifuged at 3500 g for 15 min then transferred to an analysis plate. A calibration curve in DMSO for each sample between 10 - 220 µM was prepared and included in the analysis plate. Analysis was then performed by LC-MS (Agilent Rapid Resolution HPLC, AB SCIEX 4500 MS) and solubility of each sample determined from the corresponding calibration curve.

Hepatocyte stability was performed using a 5-point assay design using cryopreserved hepatocytes in suspension. Briefly, the compounds (1 µM) were incubated at 37 °C in human (Hepatosure 100-donor pool, XenoTech LLC) and mouse (Cryostax mouse CD1, XenoTech LLC) hepatocytes (500,000 cells/ml) for various timepoints over 120 min. Reactions were quenched by adding ice-cold acetonitrile containing internal standard. The samples were then centrifuged, and the samples analysed by LC-MS/MS (Agilent Rapid Resolution HPLC, AB SCIEX 4500 MS) for the disappearance of parent compound. Half-life, clearance and hepatic excretion ratios were determined using standard equations.

Plasma protein binding was determined by ultracentrifugation. In brief, pooled human plasma was spiked with test compound (10 µM) from a 10mM DMSO stock. An aliquot was immediately removed and quenched using ice cold acetonitrile containing internal standard (carbamazepine, 0.0236 µg/mL), and placed in the freezer. This served as the total concentration sample. After preincubation (37 °C for 1 hour) duplicate aliquots of the spiked plasma were transferred to ultra-centrifugation tubes, and ultracentrifuged for 4 hours (42000 rpm, 37 °C, Beckman Optima L-80XP). Aliquots were also taken after preincubation, quenched and stored at 4°C. All samples were analyzed by LC-MS/MS (Agilent Rapid Resolution HPLC, AB SCIEX 4500 MS). Protein binding was then calculated by comparing analyte:peak area ratios of the ultracentrifuged sample to those of the total concentration sample. The percent degradation was calculated by comparing the analyte:peak area ratios of the preincubation relative to the total concentration sample. Plasma binding results were rejected if degradation was >15%.

### 3.2 Solubility test results

The high solubility of **Bsy1-6** (Table 4) in aqueous conditions at pH 7.4 provides confidence that other assay measurements performed under similar conditions would provide reproducible data, since the compound remains in solution. This solubility should, make the oral absorption process during *in vivo* experiments easier and more reproducible. However, thermodynamic solubility, which requires a solid sample of the compound would be recommended once the team starts plans for *in vivo* evaluation.

**Table 4: Solubility tests.**

| **Compound** | **Solubility (pH 7.4) (µM)** |
|---|---|
| **Finasteride** | >200 |
| **Bsy1** | >200 |
| **Bsy2** | 195 |
| **Bsy3** | 190 |
| **Bsy4** | >200 |
| **Bsy5** | >200 |
| **Bsy6** | 120 |

### 3.2 Hepatocyte stability

The hepatocyte intrinsic clearance, CL_{int,app} is a measure of how quickly a compound is metabolized in hepatocytes - a low value, as in this case, is ideal since it suggests that the compounds are metabolized very slowly. The CL_{int,app} value can be used to calculate a predicted *in vivo* value by factoring in scaling factors (number of hepatocytes per gram of liver and grams of liver per kg body weight) and binding (measured plasma protein binding and estimated incubational binding). The predicted mouse and human clearance values represent the total hepatic clearances that would be expected for these compounds *in vivo.* **Bsy1** and Finasteride would be classified as low hepatic clearance compounds, since the predicted clearances are below 30% of the hepatic blood flow in each of the species (mouse liver blood flow = 126 ml/min/kg, human liver blood flow = 20.7 ml/min/kg) (Table 5).

**Table 5: The metabolism of Bsy1 and finasteride in mouse and human hepatocytes.**

| **Compound** | **Mouse PPB (%)** | **Mouse hepatocyte stability** | | **Human hepatocyte stability** | |
|---|---|---|---|---|---|
| | | CL_{int, app} (µl/min/e6 cells) | Predicted mouse Clearance (ml/min/kg) | CL_{int, app} (µl/min/e6 cells) | Predicted human Clearance (ml/min/kg) |
| **Bsy1** | 5 | <6.4 | <13.8 | 4.1 | 6.7 |
| Finasteride | 73 | 13.8 | 10.6 | 4.5 | 3.3 |

This above description of some of the illustrative embodiments of the invention is to indicate how the invention can be made and carried out.

## Claims

1. A pharmaceutical composition comprising a compound of the Formula (I), or a pharmaceutically acceptable salt, hydrate, or solvate thereof, and a pharmaceutically acceptable carrier, 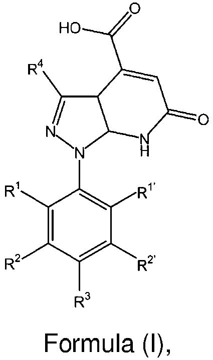 wherein,
R¹, R^{1'}, R², R^{2'}, and R³ are each independently selected from the group consisting of H, halogen, and -OR⁵,
R⁵ is selected from substituted or unsubstituted C₁-C₂alkyl, substituted or unsubstituted, linear or branched C₃-C₆alkyl, and
R⁴ is selected from substituted or unsubstituted C₁-C₂alkyl, substituted or unsubstituted, linear or branched C₃-C₆alkyl, and substituted or unsubstituted C₃-C₆cycloalkyl.

2. The pharmaceutical composition according to claim 1, wherein R¹, R^{1'}, R², and R^{2'} are H, and R³ is -OR⁵.

3. The pharmaceutical composition according to claim 1, wherein one of R¹ or R^{1'} is -OR⁵, and R², R^{2'}, and R³ are H.

4. The pharmaceutical composition according to claim 1, wherein one of R² or R^{2'} is -OR⁵, and R¹, R^{1'}, and R³ are H.

5. The pharmaceutical composition according to claim 1, wherein at least one of R¹, R^{1'}, R², R^{2'}, and R³ are halogen.

6. The pharmaceutical composition according to claim 1, wherein one of R¹, R^{1'}, R², R^{2'}, and R³ is halogen and the rest if H.

7. The pharmaceutical composition according to claim 1, wherein the halogen is selected from CI, Br, and F.

8. The pharmaceutical composition according to claim 1, wherein R⁴ is selected from unsubstituted C₁-C₂alkyl, unsubstituted, linear or branched C₃-C₆alkyl, and unsubstituted C₃-C₆cycloalkyl.

9. The pharmaceutical composition according to claim 1, wherein R⁴ is methyl, ethyl, or cyclopropyl.

10. The pharmaceutical composition according to claim 1, wherein -OR⁵ is - OCH₃.

11. The pharmaceutical composition according to claim 1, wherein the compound of the Formula (I) is selected from:
| **Compound** | **Chemical structure** | **Compound name** |
|---|---|---|
| **Bsy1** | 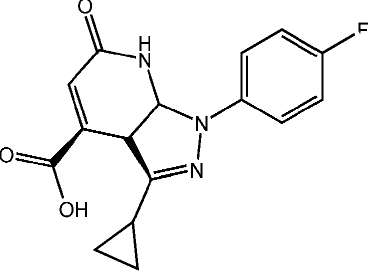 | 3-cyclopropyl-1-(4-fluorophenyl)-6-oxo-3a,6,7,7a-tetrahydro-1H-pyrazolo[3,4-b]pyridine-4-carboxylic acid |
| **Bsy2** | 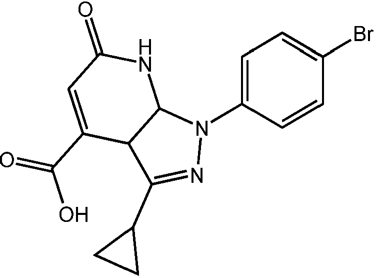 | 1-(4-bromophenyl)-3-cyclopropyl-6-oxo-3a,6,7,7a-tetrahydro-1H-pyrazolo[3,4-b]pyridine-4-carboxylic acid |
| **Bsy3** | 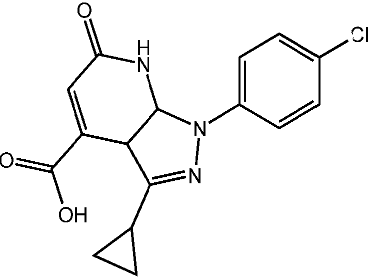 | 1-(4-chlorophenyl)-3-cyclopropyl-6-oxo-3a,6,7,7a-tetrahydro-1H-pyrazolo[3,4-b]pyridine-4-carboxylic acid |
| **Bsy4** | 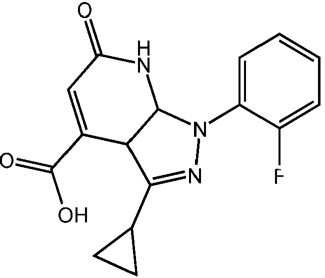 | 3-cyclopropyl-1-(2-fluorophenyl)-6-oxo-3a,6,7,7a-tetrahydro-1H-pyrazolo[3,4-b]pyridine-4-carboxylic acid |
| **Bsy5** | 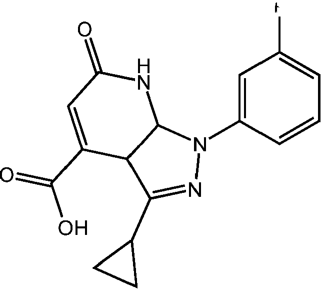 | 3-cyclopropyl-1-(3-fluorophenyl)-6-oxo-3a,6,7,7a-tetrahydro-1H-pyrazolo[3,4-b]pyridine-4-carboxylic acid |
| **Bsy6** | 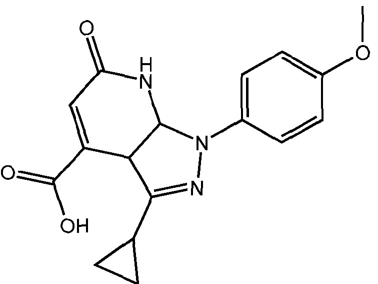 | 3-cyclopropyl-1-(4-methoxyphenyl)-6-oxo-3a,6,7,7a-tetrahydro-1H-pyrazolo[3,4-b]pyridine-4-carboxylic acid |
| **Bsy7** | 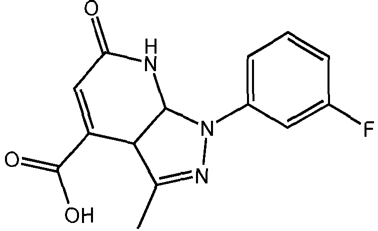 | 1-(3-fluorophenyl)-3-methyl-6-oxo-3a,6,7,7a-tetrahydro-1H-pyrazolo[3,4-b]pyridine-4-carboxylic acid |
| **Bsy8** | 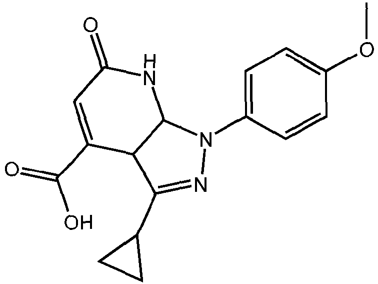 | 3-cyclopropyl-1-(4-methoxyphenyl)-6-oxo-3a,6,7,7a-tetrahydro-1H-pyrazolo[3,4-b]pyridine-4-carboxylic acid |
| **Bsy9** | 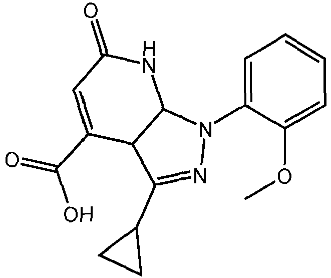 | 3-cyclopropyl-1-(2-methoxyphenyl)-6-oxo-3a,6,7,7a-tetrahydro-1H-pyrazolo[3,4-b]pyridine-4-carboxylic acid |
| **Bsy10** | 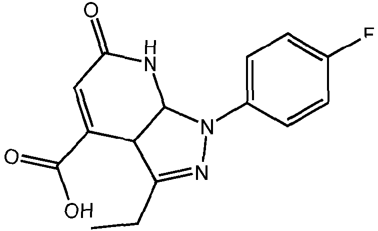 | 3-ethyl-1-(4-fluorophenyl)-6-oxo-3a,6,7,7a-tetrahydro-1H-pyrazolo[3,4-b]pyridine-4-carboxylic acid |

12. A compound of the Formula (I), or a pharmaceutically acceptable salt, hydrate, or solvate thereof, or a pharmaceutical composition comprising the compound of the Formula (I), or a pharmaceutically acceptable salt, hydrate, or solvate thereof, according to any one of claims 1 - 11, for use in treating a disease or a disorder.

13. The compound or pharmaceutical composition for use according to claim 12, wherein the compound of the Formula (I) is selected from:
| **Compound** | **Chemical structure** | **Compound name** |
|---|---|---|
| **Bsy1** | 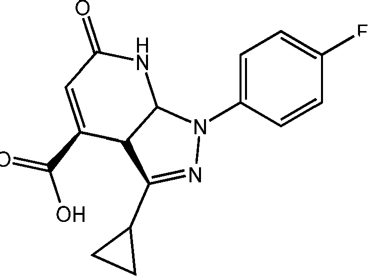 | 3-cyclopropyl-1-(4-fluorophenyl)-6-oxo-3a,6,7,7a-tetrahydro-1H-pyrazolo[3,4-b]pyridine-4-carboxylic acid |
| **Bsy2** | 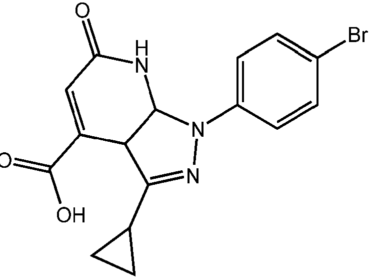 | 1-(4-bromophenyl)-3-cyclopropyl-6-oxo-3a,6,7,7a-tetrahydro-1H-pyrazolo[3,4-b]pyridine-4-carboxylic acid |
| **Bsy3** | 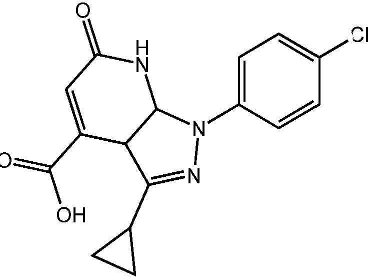 | 1-(4-chlorophenyl)-3-cyclopropyl-6-oxo-3a,6,7,7a-tetrahydro-1H-pyrazolo[3,4-b]pyridine-4-carboxylic acid |
| **Bsy4** | 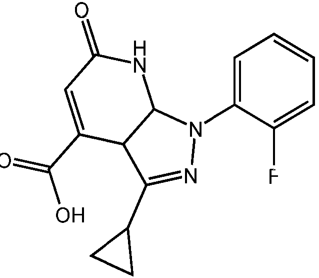 | 3-cyclopropyl-1-(2-fluorophenyl)-6-oxo-3a,6,7,7a-tetrahydro-1H-pyrazolo[3,4-b]pyridine-4-carboxylic acid |
| **Bsy5** | 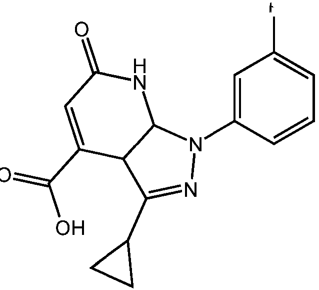 | 3-cyclopropyl-1-(3-fluorophenyl)-6-oxo-3a,6,7,7a-tetrahydro-1H-pyrazolo[3,4-b]pyridine-4-carboxylic acid |
| **Bsy6** | 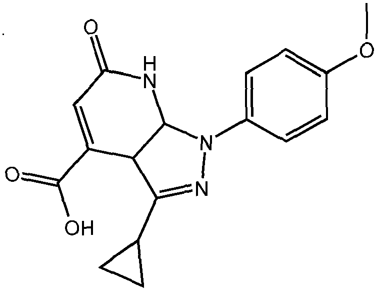 | 3-cyclopropyl-1-(4-methoxyphenyl)-6-oxo-3a,6,7,7a-tetrahydro-1H-pyrazolo[3,4-b]pyridine-4-carboxylic acid |
| **Bsy7** | 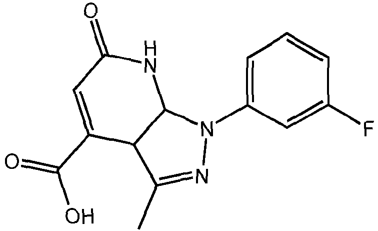 | 1-(3-fluorophenyl)-3-methyl-6-oxo-3a,6,7,7a-tetrahydro-1H-pyrazolo[3,4-b]pyridine-4-carboxylic acid |
| **Bsy8** | 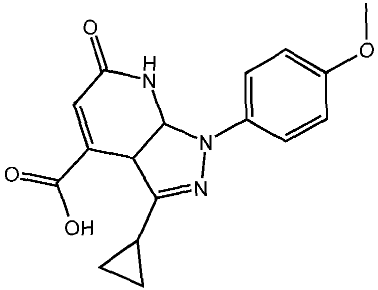 | 3-cyclopropyl-1-(4-methoxyphenyl)-6-oxo-3a,6,7,7a-tetrahydro-1H-pyrazolo[3,4-b]pyridine-4-carboxylic acid |
| **Bsy9** | 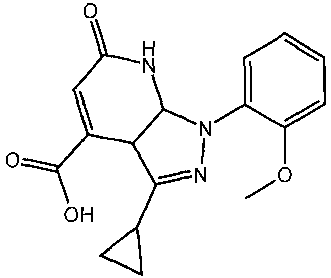 | 3-cyclopropyl-1-(2-methoxyphenyl)-6-oxo-3a,6,7,7a-tetrahydro-1H-pyrazolo[3,4-b]pyridine-4-carboxylic acid |
| **Bsy10** | 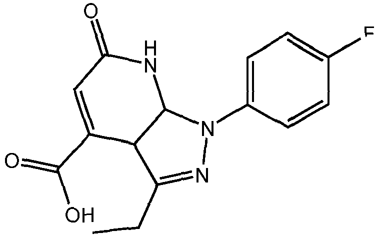 | 3-ethyl-1-(4-fluorophenyl)-6-oxo-3a,6,7,7a-tetrahydro-1H-pyrazolo[3,4-b]pyridine-4-carboxylic acid |

14. The compound or pharmaceutical composition for use, according to claim 12 or claim 13 wherein, the disease or disorder is benign prostatic hyperplasia.

15. The compound or pharmaceutical composition for use, according to claim 12 or claim 13 wherein, the disease or disorder is androgenic alopecia.

## Patentansprüche

1. Pharmazeutische Zusammensetzung, umfassend eine Verbindung der Formel (I) oder ein pharmazeutisch verträgliches Salz, Hydrat oder Solvat davon, und einen pharmazeutisch verträglichen Träger, 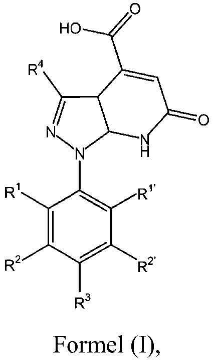 wobei
R¹, R ^{1'}, R², R^{2'} und R³ jeweils unabhängig voneinander aus der Gruppe bestehend aus H, Halogen und -OR⁵ ausgewählt sind,
R⁵ aus substituiertem oder unsubstituiertem C₁-C₂-Alkyl, substituiertem oder unsubstituiertem, linearem oder verzweigtem C₃-C₆-Alkyl ausgewählt ist, und
R⁴ aus substituiertem oder unsubstituiertem C₁-C₂-Alkyl, substituiertem oder unsubstituiertem, linearem oder verzweigtem C₃-C₆-Alkyl und substituiertem oder unsubstituiertem C₃-C₆-Cycloalkyl ausgewählt ist.

2. Pharmazeutische Zusammensetzung nach Anspruch 1, wobei R¹, R ^{1'}, R² und R^{2'} H sind, und R³ -OR⁵ ist.

3. Pharmazeutische Zusammensetzung nach Anspruch 1, wobei eines von R¹ oder R ^{1'} -OR⁵ ist und R², R^{2'} und R³ H sind.

4. Pharmazeutische Zusammensetzung nach Anspruch 1, wobei eines von R² oder R ^{2'} -OR⁵ ist und R¹, R^{1'} und R³ H sind.

5. Pharmazeutische Zusammensetzung nach Anspruch 1, wobei mindestens eines von R¹, R^{1'}, R², R^{2'}, und R³ Halogen ist.

6. Pharmazeutische Zusammensetzung nach Anspruch 1, wobei eines von R¹, R^{1'}, R², R^{2'}, und R³ Halogen ist, und der Rest H ist.

7. Pharmazeutische Zusammensetzung nach Anspruch 1, wobei das Halogen aus Cl, Br und F ausgewählt ist.

8. Pharmazeutische Zusammensetzung nach Anspruch 1, wobei R⁴ aus unsubstituiertem C₁-C₂-Alkyl, unsubstituiertem, linearem oder verzweigtem C₃-C₆-Alkyl und unsubstituiertem C₃-C₆-Cycloalkyl ausgewählt ist.

9. Pharmazeutische Zusammensetzung nach Anspruch 1, wobei R⁴ Methyl, Ethyl oder Cyclopropyl ist.

10. Pharmazeutische Zusammensetzung nach Anspruch 1, wobei -OR⁵ -OCH₃ ist.

11. Pharmazeutische Zusammensetzung nach Anspruch 1, wobei die Verbindung der Formel (I) ausgewählt ist aus:
| **Verbindung** | **Chemische Struktur** | **Name der Verbindung** |
|---|---|---|
| **Bsy1** | 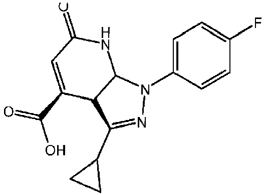 | 3-Cyclopropyl-1-(4-fluorphenyl)-6-oxo-3a,6,7,7a-tetrahydro-1H-pyrazol[3,4-b]pyridin-4-carbonsäure |
| **Bsy2** | 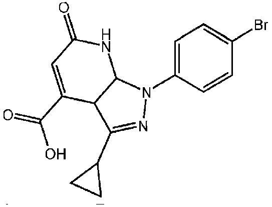 | 1-(4-Bromphenyl)-3-cyclopropyl-6-oxo-3a,6,7,7a-tetrahydro-1H-pyrazol[3,4-b]pyridin-4-carbonsäure |
| **Bsy3** | 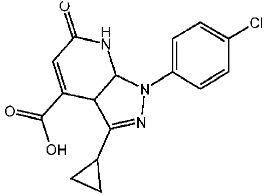 | 1-(4-Chlorphenyl)-3-cyclopropyl-6-oxo-3a,6,7,7a-tetrahydro-1H-pyrazol[3,4-b]pyridin-4-carbonsäure |
| **Bsy4** | 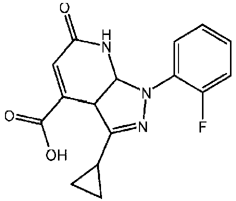 | 3-Cyclopropyl-1-(2-Fluorphenyl)-6-oxo-3a,6,7,7a-tetrahydro-1H-pyrazol[3,4-b]pyridin-4-carbonsäure |
| **Bsy5** | 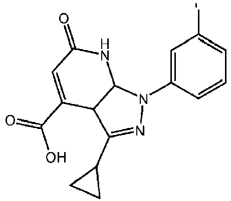 | 3-Cyclopropyl-1-(3-fluorphenyl)-6-oxo-3a,6,7,7a-tetrahydro-1H-pyrazol[3,4-b]pyridin-4-carbonsäure |
| **Bsy6** | 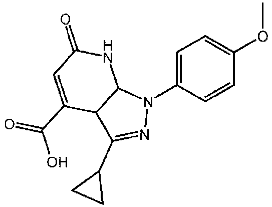 | 3-Cyclopropyl-1-(4-Methoxyphenyl)-6-oxo-3a,6,7,7a-tetrahydro-1H-pyrazol[3,4-b]pyridin-4-carbonsäure |
| **Bsy7** | 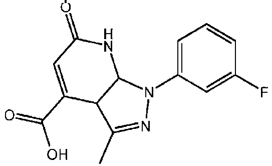 | 1-(3-Fluorphenyl)-3-methyl-6-oxo-3a,6,7,7a-tetrahydro-1H-pyrazol[3,4-b]pyridin-4-carbonsäure |
| **Bsy8** | 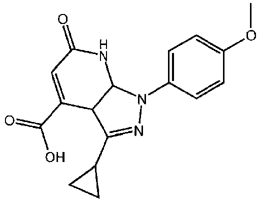 | 3-Cyclopropyl-1-(4-Methoxyphenyl)-6-oxo-3a,6,7,7a-tetrahydro-1H-pyrazol[3,4-b]pyridin-4-carbonsaure |
| **Bsy9** | 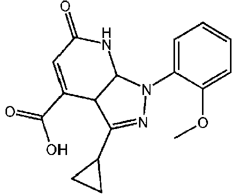 | 3-Cyclopropyl-1-(2-methoxyphenyl)-6-oxo-3a,6,7,7a-tetrahydro-1H-pyrazol[3,4-b]pyridin-4-carbonsäure |
| **Bsy10** | 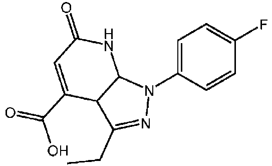 | 3-Ethyl-1-(4-fluorphenyl)-6-oxo-3a,6,7,7a-tetrahydro-1H-pyrazol[3,4-b]pyridin-4-carbonsäure |

12. Verbindung der Formel (I) oder pharmazeutisch verträgliches Salz, Hydrat oder Solvat davon oder pharmazeutische Zusammensetzung, umfassend die Verbindung der Formel (I) oder ein pharmazeutisch verträgliches Salz, Hydrat oder Solvat davon nach einem der Ansprüche 1 - 11 zur Verwendung bei der Behandlung einer Krankheit oder Störung.

13. Verbindung oder pharmazeutische Zusammensetzung zur Verwendung nach Anspruch 12, wobei die Verbindung der Formel (I) ausgewählt ist aus:
| **Verbindung** | **Chemische Struktur** | **Name der Verbindung** |
|---|---|---|
| **Bsy1** | 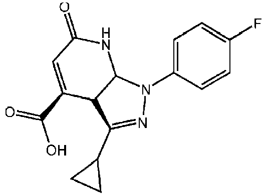 | 3-Cyclopropyl-1-(4-fluorphenyl)-6-oxo-3a,6,7,7a-tetrahydro-1H-pyrazol[3,4-b]pyridin-4-carbonsäure |
| **Bsy2** | 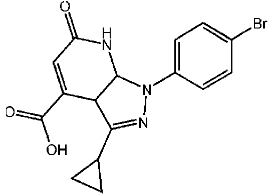 | 1-(4-Bromphenyl)-3-cyclopropyl-6-oxo-3a,6,7,7a-tetrahydro-1H-pyrazol[3,4-b]pyridin-4-carbonsäure |
| **Bsy3** | 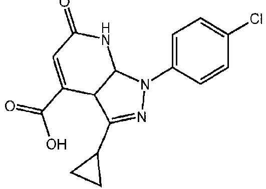 | 1-(4-Chlorphenyl)-3-cyclopropyl-6-oxo-3a,6,7,7a-tetrahydro-1H-pyrazol[3,4-b]pyridin-4-carbonsäure |
| **Bsy4** | 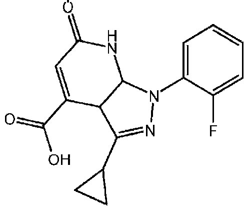 | 3-Cyclopropyl-1-(2-Fluorphenyl)-6-oxo-3a,6,7,7a-tetrahydro-1H-pyrazol[3,4-b]pyridin-4-carbonsäure |
| **Bsy5** | 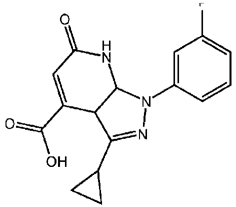 | 3-Cyclopropyl-1-(3-fluorphenyl)-6-oxo-3a,6,7,7a-tetrahydro-1H-pyrazol[3,4-b]pyridin-4-carbonsäure |
| **Bsy6** | 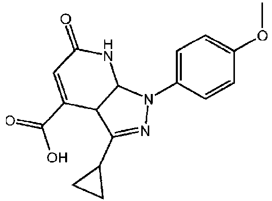 | 3-Cyclopropyl-1-(4-Methoxyphenyl)-6-oxo-3a,6,7,7a-tetrahydro-1H-pyrazol[3,4-b]pyridin-4-carbonsäure |
| **Bsy7** | 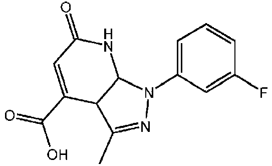 | 1-(3-Fluorphenyl)-3-methyl-6-oxo-3a,6,7,7a-tetrahydro-1H-pyrazol[3,4-b]pyridin-4-carbonsäure |
| **Bsy8** | 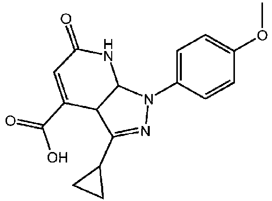 | 3-Cyclopropyl-1-(4-Methoxyphenyl)-6-oxo-3a,6,7,7a-tetrahydro-1H-pyrazol[3,4-b]pyridin-4-carbonsäure |
| **Bsy9** | 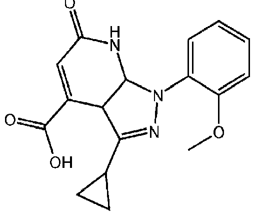 | 3-Cyclopropyl-1-(2-methoxyphenyl)-6-oxo-3a,6,7,7a-tetrahydro-1H-pyrazol[3,4-b]pyridin-4-carbonsäure |
| **Bsy10** | 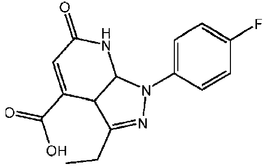 | 3-Ethyl-1-(4-fluorphenyl)-6-oxo-3a,6,7,7a-tetrahydro-1H-pyrazol[3,4-b]pyridin-4-carbonsäure |

14. Verbindung oder pharmazeutische Zusammensetzung zur Verwendung nach Anspruch 12 oder Anspruch 13, wobei die Krankheit oder Störung eine benigne Prostatahyperplasie ist.

15. Verbindung oder pharmazeutische Zusammensetzung zur Verwendung nach Anspruch 12 oder Anspruch 13, wobei die Krankheit oder Störung androgene Alopezie ist.

## Revendications

1. Composition pharmaceutique comprenant un composé de la Formule (I), ou un sel, un hydrate ou un solvant pharmaceutiquement acceptable de celui-ci, et un excipient pharmaceutiquement acceptable, 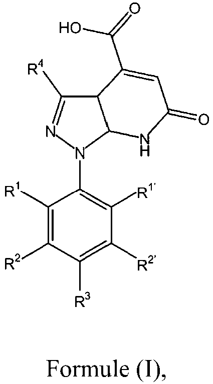 dans laquelle :
R¹, R^{1'}, R², R^{2'} et R³ sont indépendamment choisis parmi le groupe constitué par H, un halogène et -OR⁵
R⁵ est choisi parmi les groupes alkyle en C₁-C₂ substitués ou non substitués, alkyle linéaire ou ramifié en C₃-C₆ substitués ou non substitués, et
R⁴ est choisi parmi les groupes alkyle en C₁-C₂ substitués ou non substitués, alkyle linéaire ou ramifié en C₃-C₆ substitués ou non substitués, et cycloalkyle en C₃-C₆ substitué ou non substitué.

2. Composition pharmaceutique selon la revendication 1, dans laquelle R¹, R^{1'}, R² et R^{2'} sont H, et R³ est -OR⁵

3. Composition pharmaceutique selon la revendication 1, dans laquelle l'un de R¹ ou R^{1'} est -OR⁵, et R², R^{2'} et R³ sont H.

4. Composition pharmaceutique selon la revendication 1, dans laquelle l'un de R² ou R^{2'} est -OR⁵, et R¹, R^{1'} et R³ sont H.

5. Composition pharmaceutique selon la revendication 1, dans laquelle au moins l'un de R¹, R^{1'}, R², R^{2'} et R³ est un atome d'halogène.

6. Composition pharmaceutique selon la revendication 1, dans laquelle l'un de R¹, R^{1'}, R², R^{2'} et R³ est un atome d'halogène et les autres sont H.

7. Composition pharmaceutique selon la revendication 1, dans laquelle l'halogène est choisi parmi Cl, Br et F.

8. Composition pharmaceutique selon la revendication 1, dans laquelle R⁴ est choisi parmi les groupes alkyle en C₁-C₂ non substitués, alkyle linéaire ou ramifié en C₃-C₆ non substitué, et cycloalkyle en C₃-C₆ non substitué.

9. Composition pharmaceutique selon la revendication 1, dans laquelle R⁴ est le méthyle, l'éthyle ou le cyclopropyle.

10. Composition pharmaceutique selon la revendication 1, dans laquelle -OR⁵ est - OCH₃.

11. Composition pharmaceutique selon la revendication 1, dans laquelle le composé de la Formule (1) est choisi parmi :
| **Composé** | **Structure chimique** | **Nom du composé** |
|---|---|---|
| **Bsy1** | 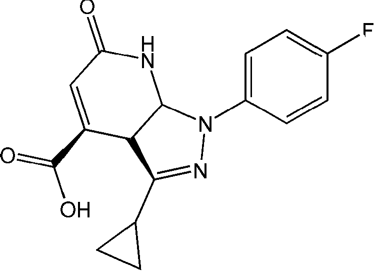 | acide 3-cyclopropyl-1-(4-fluorophényl)-6-oxo-3a,6,7,7a-tétrahydro-1H-pyrazolo [3,4-b]pyridine-4-carboxylique |
| **Bsy2** | 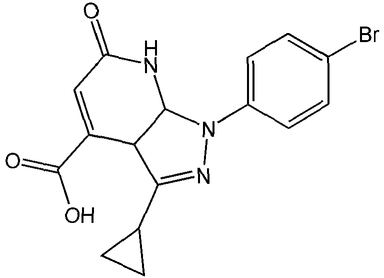 | acide 1-(4-bromophényl)-3-cyclopropyl-6-oxo-3a,6,7,7a-tetrahydro-1H-pyrazolo[3,4-b]pyridine-4-carboxylique |
| **Bsy3** | 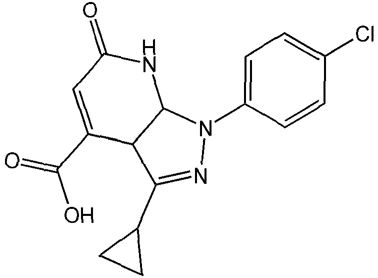 | acide 1-(4-chlorophényl)-3-cyclopropyl-6-oxo-3a,6,7,7a-tetrahydro-1H-pyrazolo[3,4-b]pyridine-4-carboxylique |
| **Bsy4** | 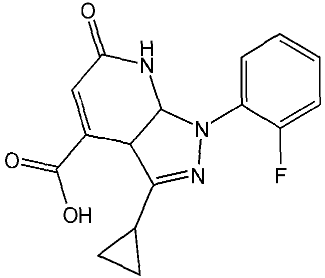 | acide 3-cyclopropyl-1-(2-fluorophényl)-6-oxo-3a,6,7,7a-tétrahydro-1H-pyrazolo[3,4-b]pyridine-4-carboxylique |
| **Bsy5** | 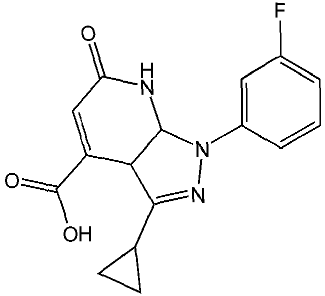 | acide 3-cyclopropyl-1-(3-fluorophényl)-6-oxo-3a,6,7,7a-tétrahydro-1H-pyrazolo [3,4-b]pyridine-4-carboxylique |
| **Bsy6** | 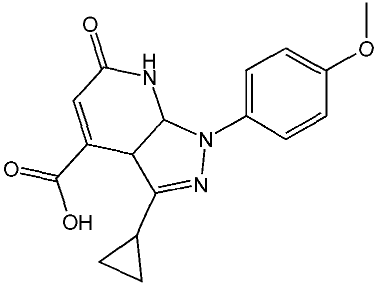 | acide 3-cyclopropyl-1-(4-méthoxyphényl)-6-oxo-3a,6,7,7a-tétrahydro-1H-pyrazolo[3,4-b]pyridine-4-carboxylique |
| **Bsy7** | 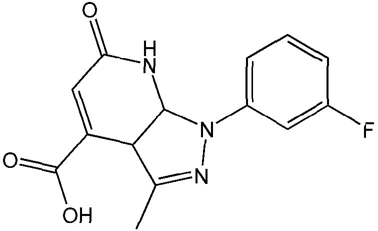 | acide 1-(3-fluorophényl)-3-méthyl-6-oxo-3a,6,7,7a-tétrahydro-1H-pyrazolo[3,4-b]pyridine-4-carboxylique |
| **Bsy8** | 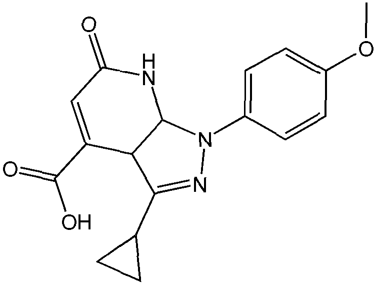 | acide 3-cyclopropyl-1-(4-méthoxyphényl)-6-oxo-3a,6,7,7a-tétrahydro-1H-pyrazolo [3,4-b]pyridine-4-carboxylique |
| **Bsy9** | 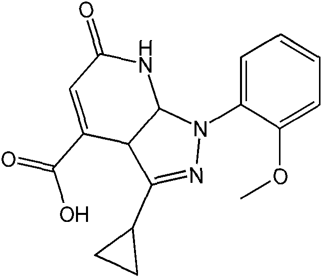 | acide 3-cyclopropyl-1-(2-méthoxyphényl)-6-oxo-3a,6,7,7a-tétrahydro-1H-pyrazolo[3,4-b]pyridine-4-carboxylique |
| **Bsy10** | 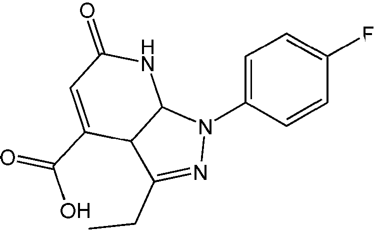 | acide 3-éthyl-1-(4-fluorophényl)-6-oxo-3a,6,7,7a-tétrahydro-1H-pyrazolo [3,4-b]pyridine-4-carboxylique |

12. Composé de la Formule (I), ou sel, hydrate ou solvant pharmaceutiquement acceptable de celui-ci, ou composition pharmaceutique comprenant ledit composé de la Formule (I), ou ledit sel, hydrate ou solvant pharmaceutiquement acceptable de celui-ci, selon l'une quelconque des revendications 1 à 11, pour l'utilisation dans le traitement d'une maladie ou d'une affection.

13. Composé ou composition pharmaceutique pour l'utilisation selon la revendication 12, dans lequel le composé de la Formule (I) est choisi parmi :
| **Composé** | **Structure chimique** | **Nom du composé** |
|---|---|---|
| **Bsy1** | 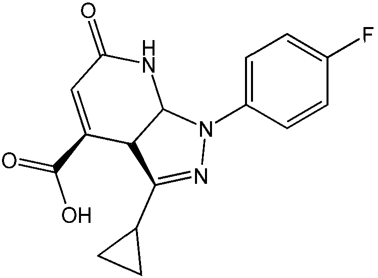 | acide 3-cyclopropyl-1-(4-fluorophenyl)-6-oxo-3a,6,7,7a-tétrahydro-1H-pyrazolo [3,4-b]pyridine-4-carboxylique |
| **Bsy2** | 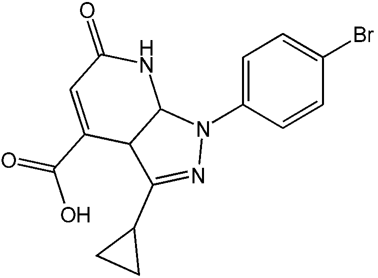 | acide 1-(4-bromophényl)-3-cyclopropyl-6-oxo-3a,6,7,7a-tétrahydro-1H-pyrazolo [3,4-b]pyridine-4-carboxylique |
| **Bsy3** | 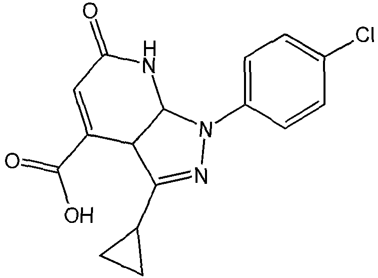 | acide 1-(4-chlorophényl)-3-cyclopropyl-6-oxo-3a,6,7,7a-tétrahydro-1H-pyrazolo[3,4-b]pyridine-4-carboxylique |
| **Bsy4** | 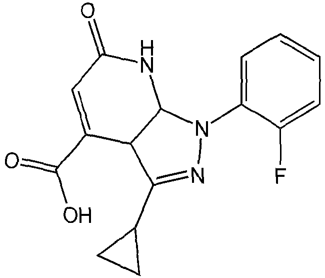 | acide 3-cyclopropyl-1-(2-fluorophényl)-6-oxo-3a,6,7,7a-tétrahydro-1H-pyrazolo [3,4-b]pyridine-4-carboxylique |
| **Bsy5** | 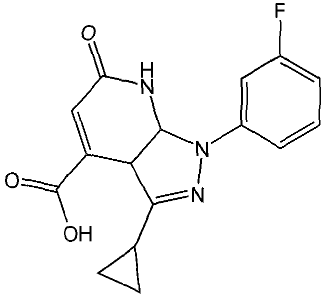 | acide 3-cyclopropyl-1-(3-fluorophényl)-6-oxo-3a,6,7,7a-tétrahydro-1H-pyrazolo[3,4-b]pyridine-4-carboxylique |
| **Bsy6** | 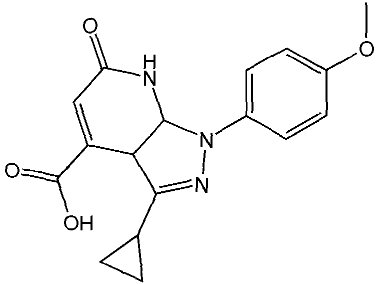 | acide 3-cyclopropyl-1-(4-méthoxyphényl)-6-oxo-3a,6,7,7a-tétrahydro-1H-pyrazolo[3,4-b]pyridine-4-carboxylique |
| **Bsy7** | 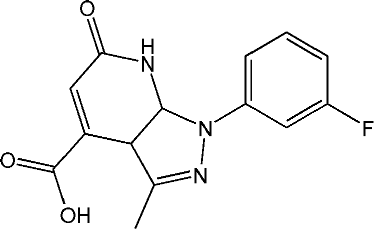 | acide 1-(3-fluorophényl)-3-méthyl-6-oxo-3a,6,7,7a-tétrahydro-1H-pyrazolo[3,4-b]pyridine-4-carboxylique |
| **Bsy8** | 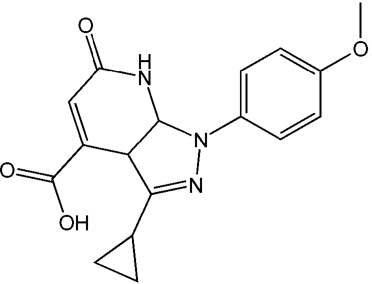 | acide 3-cyclopropyl-1-(4-méthoxyphényl)-6-oxo-3a,6,7,7a-tétrahydro-1H-pyrazolo [3,4-b]pyridine-4-carboxylique |
| **Bsy9** | 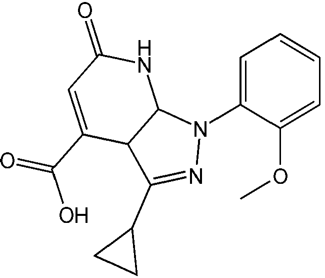 | acide 3-cyclopropyl-1-(2-méthoxyphényl)-6-oxo-3a,6,7,7a-tétrahydro-1H-pyrazolo[3,4-b]pyridine-4-carboxylique |
| **Bsy10** | 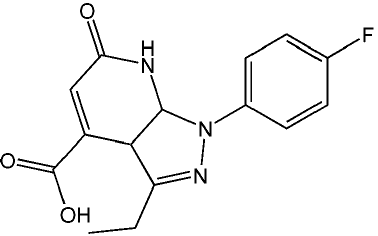 | acide 3-éthyl-1-(4-fluorophényl)-6-oxo-3a,6,7,7a-tetrahydro-1H-pyrazolo [3,4-b]pyridine-4-carboxylique |

14. Composé ou composition pharmaceutique pour l'utilisation selon la revendication 12 ou la revendication 13, dans lequel ladite maladie ou ladite affection est une hyperplasie bénigne de la prostate.

15. Composé ou composition pharmaceutique pour l'utilisation selon la revendication 12 ou la revendication 13, dans lequel ladite maladie ou ladite affection est une alopécie androgénétique.
